(19) Europäisches Patentamt
European Patent Office
Office européen des brevets

(11) Veröffentlichungsnummer: **0 107 624**
**B1**

(12) # EUROPÄISCHE PATENTSCHRIFT

(45) Veröffentlichungstag der Patentschrift: **20.05.87**

(21) Anmeldenummer: **83810482.6**

(22) Anmeldetag: **19.10.83**

(51) Int. Cl.⁴: **C 07 D 239/42**, C 07 D 239/47, C 07 D 251/46, C 07 C 143/78, C 07 C 149/443 // A01N47/36

(54) **Verfahren zur Herstellung von herbizid wirksamen und den Pflanzenwuchs regulierenden Sulfonylharnstoffen.**

(30) Priorität: **25.10.82 CH 6202/82**

(43) Veröffentlichungstag der Anmeldung: **02.05.84 Patentblatt 84/18**

(45) Bekanntmachung des Hinweises auf die Patenterteilung: **20.05.87 Patentblatt 87/21**

(84) Benannte Vertragsstaaten: **BE CH DE FR GB IT LI NL**

(56) Entgegenhaltungen:
**EP-A-0 023 422**
**EP-A-0 024 215**
**EP-A-0 044 807**
**EP-A-0 044 808**
**DE-B-1 215 143**
**US-A-4 302 241**

(73) Patentinhaber: **CIBA- GEIGY AG, Klybeckstrasse 141, CH- 4002 Basel (CH)**

(72) Erfinder: **Kristinsson, Haukur, Dr., Kreuzackerweg 19, CH- 4103 Bottmingen (CH)**
Erfinder: **Töpfl, Werner, Dr., Dorneckstrasse 68, CH- 4143 Dornach (CH)**

**EP 0 107 624 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft ein neues Verfahren zur Herstellung von herbizid wirksamen und den Pflanzenwuchs regulierenden Sulfonylharnstoffen sowie neue als Zwischenprodukte hergestellte Sulfonylimido-Kohlensäureester und N-Sulfonyl-N'-triazinyl- oder pyrimidinylisoharnstoffe.

Die Sulfonylharnstoffe entsprechen der allgemeinen Formel I

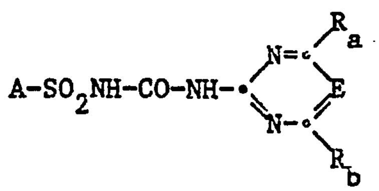

(I) ,

worin

A einen Rest der Formeln

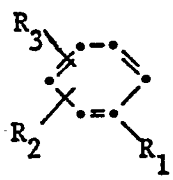 , 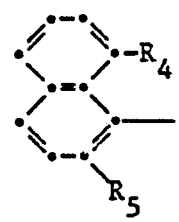 oder 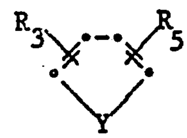 ,

Y Sauerstoff Schwefel oder 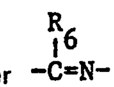 ,

$R_1$ Wasserstoff, Halogen, Nitro, Trifluormethyl, $C_1$-$C_5$ Alkyl, $C_1$-$C_5$ Alkoxy, $-COR_7$, $-S(O)_m$ $C_1$-$C_5$ Alkyl, $-SO_2R_{10}$ oder $XR_{11}$, $-OSO_2C_1$-$C_5$ Alkyl,

$R_2$ Wasserstoff, Fluor, Chlor, Brom, Nitro, $C_1$-$C_5$ Alkyl, $C_1$ -$C_5$ Alkoxy, Trifluormethyl, $C_1$-$C_5$ Halogenalkoxy oder $-COR_7$,

$R_3$ Wasserstoff, Fluor, Chlor, Brom, Nitro, Methoxy oder Trifluormethyl,

$R_4$ Wasserstoff, Halogen, Nitro $C_1$-$C_5$ Alkyl, Methoxy, $-COR_7$ oder $-SO_2NR_8R_9$,

$R_5$ Wasserstoff, Fluor, Chlor, Brom, Nitro, $C_1$-$C_5$ Alkyl, $C_1$-$C_5$ Alkoxy, Trifluormethyl, $-S(O)_mC_1$-$C_5$ Alkyl, $-COR_7$, $-SO_2NR_8R_9$

$R_6$ Wasserstoff, Fluor, Methyl, Methoxy,

$R_7$ Wasserstoff, $C_1$-$C_5$ Alkyl, $C_1$-$C_5$ Halogenalkyl, $C_1$-$C_5$ Alkoxy, $C_1$-$C_5$ Halogenalkoxy, $C_2$-$C_{10}$ Alkoxyalkoxy, $C_3$-$C_5$ Alkenyloxy, $C_3$-$C_5$ Alkinyloxy, Phenoxy, Benzyloxy, $C_1$-$C_5$ Alkylthio oder $-NR_8R_9$

$R_8$ Wasserstoff, $C_1$-$C_5$ Alkyl, Cyanoalkyl mit höchstens 5 Kohlenstoffatomen, Methoxy, Äthoxy oder $C_3$-$C_5$ Alkenyl,

$R_9$ Wasserstoff, $C_1$-$C_5$ Alkyl oder $C_3$-$C_5$ Alkenyl, oder

$R_8$ und $R_9$ zusammen mit dem sie bindenden Stickstoffatom einen 5- bis 6-gliedrigen gesättigten, gegebenenfalls ein Sauerstoff- oder Schwefelatom als Ringglied enthaltenden, Heterocyclus,

$R_{10}$ $C_1$-$C_5$ Halogenalkoxy oder $-NR_8R_9$,

$R_{11}$ $C_1$-$C_5$ Alkyl welches substituiert ist durch Halogen, $C_1$-$C_5$ Alkoxy, $-S(O)_m$ $C_1$-$C_5$ Alkyl, $-S(O)_mC_1$-$C_5$ Halogenalkyl oder $C_2$-$C_5$ Alkenyl welches gegebenenfalls durch einen der aufgezählten Reste substituiert ist,

X Sauerstoff oder $-S(O)_m$-,

m eine Zahl von Null, Eins oder Zwei,

E die Methingruppe oder Stickstoff,

$R_a$ Wasserstoff, Halogen, $C_1$-$C_5$ Alkyl, $C_1$-$C_5$ Halogenalkyl, $C_1$-$C_5$ Alkoxy, $C_1$-$C_5$ Halogenalkoxy, $C_1$-$C_5$ Alkylthio, $C_2$-$C_{10}$ Alkoxyalkyl oder $C_2$-$C_{10}$ Alkoxyalkoxy,

$R_b$ dasselbe wie $R_a$ oder eine Aminogruppe 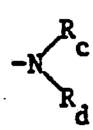 , worin

$R_c$ Wasserstoff; Methyl oder Äthyl und

$R_d$ Wasserstoff, Methyl, Äthyl oder Methoxy,

bedeutet sowie die Salze dieser Verbindungen.

Unter Halogen ist im Rahmen der obigen Definition im allgemeinen Fluor, Chlor, Brom oder Jod zu verstehen. Bevorzugt sind Fluor und Chlor.

Beispiele für Alkyl sind Methyl, Äthyl, n-Propyl, i-Propyl oder die isomeren Butyle. Alkyl ist dabei selbst als Substituent oder als Teil eines anderen Substituenten wie beispielsweise Alkoxy oder Alkylthio zu verstehen. Bevorzugte Alkylreste sind jeweils unverzweigte Alkylketten, insbesondere aber Methyl und Äthyl.

In der Regel sind unter Alkenyl, Allyl, 2-Butenyl, 3-Butenyl, 2-Isobutenyl, Isopropenyl, 2-Pentenyl, 3-Pentenyl und 4-Pentenyl, insbesondere aber Allyl und 4-Pentenyl zu verstehen.

Unter Alkinyl versteht man im allgemeinen Propargyl, 2-Butinyl, 3-Butinyl, Methylpropargyl, 2-Pentinyl, 3-Pentinyl und 4-Pentinyl.

Die Heterocyclen, die unter die Definition des Restes A fallen, sind Thiophen, Furan, und Pyridin.

Die nach dem neuen erfindungsgemässen Verfahren herzustellenden Sulfonylharnstoffe sind z.B. in den US-Patenten 4 301 286 und 4 302 241, den publizierten europäischen Patentanmeldungen 23 422, 44 807 und 44 808 oder der deutschen Offenlegungsschrift 2 715 786 mit ihren Eigenschaften beschrieben.

Bisher wurden diese Sulfonylharnstoffe entweder durch Umsetzung eines entsprechenden Sulfonylisocyanates mit einem entsprechenden Amin oder durch Umsetzung eines Sulfonylamides mit einem, vom jeweils zu verwendenden Amin abgeleiteten Carbaminsäureester, insbesondere einem Phenylcarbamat, hergestellt.

Die bekannten Verfahren sind insofern nachteilig, als entweder mit Isocyanat- oder Isothiocyanat-Derivaten gearbeitet werden muss, deren Handhabung wegen der grossen Reaktivität dieser Verbindungsklasse schwierig ist, oder bei der Synthese aus Phenylcarbamaten ökologisch belastende Nabenprodukte wie z.B. Phenole anfallen.

Dazu kommt, dass eine Reihe von ortho-substituierten Arylsulfonylaniden schwer oder nicht herstellbar sind. Bei diesem Verfahren sind Sulfonamide nicht Zwischenprodukte.

Es ist somit Ziel der vorliegenden Erfindung, ein Verfahren bereitzustellen, das die Verwendung schlecht handhabbarer oder schwer herstellbarer Verbindungen und den Anfall von umweltbelastenden Nebenprodukten vermeidet.

Erfindungsgemäss wird somit vorgeschlagen, die herbiziden und den Pflanzenwuchsregulierenden Sulfonylharnstoffe entsprechend der Formel I gemäss einem neuen Verfahren herzustellen.

Das erfindungsgemässe Verfahren verläuft in mehreren Stufen. In der ersten Stufe wird ein Sulfochlorid der Formel II

A - $SO_2Cl$ (II),

worin A die oben gegebene Bedeutung hat, in einem inerten Phasensystem oder Lösungsmittel, in Gegenwart der mindestens äquimolaren Menge einer Base als säurebindendes Mittel, bei einer Temperatur, die zwischen 0 und 100°C liegt, mit einem Imidokohlensäureester der Formel III

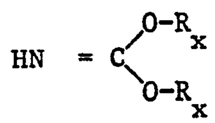 (III),

worin $R_x$ $C_1$-$C_5$ Alkyl bedeutet, kondensiert, wobei ein Sulfonylimido-Kohlensäurediester der Formel IV entsteht

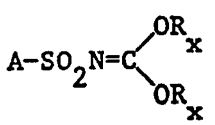 (IV) ,

worin A und $R_x$ die oben gegebene Bedeutung haben.

Die Imidokohlensäureester der Formel III sind bekannt, siehe z.B. Liebigs Ann. Chem. 287, 310 (1895) und Chem. Ber. 19, 862 (1886).

Einige N-Benzolsulfonylimid-Kohlensäureester entsprechend der Formel IV (diejenigen, worin A den Phenyl-, para Tolyl- oder para Chlorphenylrest bedeutet) sind bekannt und wurden aus dem entsprechenden Benzolsulfonylamid hergestellt, (siehe Arch. Pharmaz. 300 (1967) 553, J. Org. Chem. 28 (1963) 2902 und Chem. Ber. 99 (1966) 2200.

Der erfindungsgemässe Syntheseweg ist neu und ermöglicht die Herstellung der Sulfonylimido-Kohlensäureester der Formel IV direkt aus dem Sulfonylchlorid unter Umgehung des Sulfonamides. Die erfindungsgemässen Sulfonylimido-Kohlensäureester der Formel IV sind neue Verbindungen.

Als Lösungsmittel kommen für diese Verfahrensstufe Kohlenwasserstoffe wie Benzol, Toluol und Xylol; Äther wie Diäthyläther, Äthylenglykoldimethyläther, Diäthylenglykoldimethyläther, Tetrahydrofuran und Dioxan; Ketone wie Aceton, Äthylmethylketon und Cyclohexanon; Nitrile wie Acetonitril und Propionitril; und Dimethylsulfoxid. Die Umsetzung erfolgt in Gegenwart von mindestens äquimolaren Mengen einer Base oder in einem mindestens molaren Überschuss des zu verwendenden Iminoderivates. Geeignete Basen sind Carbonate wie Natrium-und Kaliumcarbonat, Hydrogencarbonate wie Natrium- und Kaliumhydrogencarbonat,

Oxide wie Calcium- und Magnesiumoxid und tertiäre Amine wie Trimethylamin, Triäthylamin, Chinuclidin, Chinolin, Pyridin und Tripropylamin. Mit Vorteil wird die Base im Überschuss eingesetzt. So werden pro Mol Sulfonylchlorid vorzugsweise 1 bis 5 Mol Base, insbesondere 1,1 bis 1,5 Mol, eingesetzt. Grössere Basenüberschüsse werden besonders dann verwendet, wenn die Reaktion ohne Lösungsmittel durchgeführt wird und die Base, vorzugsweise ein flüssiges tertiäres Amin, gleichzeitig als Reaktionsmedium dient. Die Reaktionstemperaturen liegen zwischen 0°C und 100°C, vorzugsweise zwischen 10°C und 80°C

In der zweiten Stufe des Verfahrens wird der aus der ersten Stufe erhaltene Sulfonylimido-Kohlensäurediester der Formel IV in einem inerten Lösungsmittel, in Gegenwart einer starken Base, bei einer Temperatur zwischen 0° und 100°C, mit einem 2-Aminopyrimidin oder einem 2-Amino-1,3,5-triazin der Formel V umgesetzt

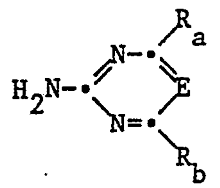 (V),

worin

E, $R_a$ und $R_b$ die oben gegebene Bedeutung haben. Dabei entsteht ein Sulfonylisoharnstoff.

Die Umsetzung des N-para Tolylsulfonylimido-Kohlensäurediäthylesters mit Pyrrolidin und n-Butylamin ist im J.Org.Chem. 28 (1963) 2902 beschrieben.

Als Lösungsmittel kommen für diesen Verfahrensschritt z.B. Diäthyläther, Dipropyläther, Dioxan, Tetrahydrofuran. Äthylenglykoldimethyläther oder Diäthylenglykoldimethyläther in Frage. Als starke Basen werden Alkalimatallhydride, Alkalimetallalkoholate, Alkalimetallalkylate, z.B. Natriumhydrid, Kalium- Butylat verwendet.

In der letzten Stufe wird schliesslich der aus der zweiten Stufe erhaltene Isoharnstoff der Formel VI

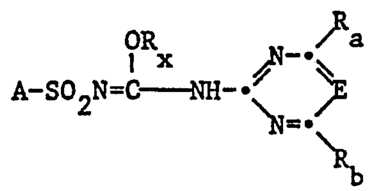 (VI) ,

worin

A, E, $R_a$, $R_b$ und $R_x$ die oben gegebene Bedeutung haben, in einem organischen Lösungsmittel in Gegenwart eines Halogenwasserstoffes und einer Temperatur von 0-100°C zum Sulfonylharnstoff der Formel I umsetzt und diesen Harnstoff als solcher oder als Salz isoliert.

Als Halogenwasserstoffe kommen insbesondere Chlor- und Bromwasserstoff im Frage. Isoharnstoffe der Formel VI sind bekannt aus der EP-A-24 215. Sie werden hergestellt durch Alkylierung der entsprechenden Sulfonylharnstoffe oder sie können auch gemäss dem folgenden Reaktionsschema hergestellt werden:

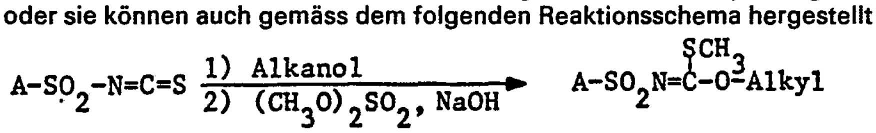


Ein durch Umsetzen von Sulfonamid mit Schwefelkohlenstoff erhaltenes Sulfonylisothiocyanat wird zuerst mit einem Alkanol dann mit Dimethylsulfat und Natronlauge zum Thiomethyl-sulfonylimido-kohlensäureester umgesetzt, welcher dann mit Sulfonylchlorid zum Sulfonylimido-kohlensäureester-monochlorid und weiter mit dem Lithiumsalz eines Amines zum Isoharnstoff umgesetzt wird.

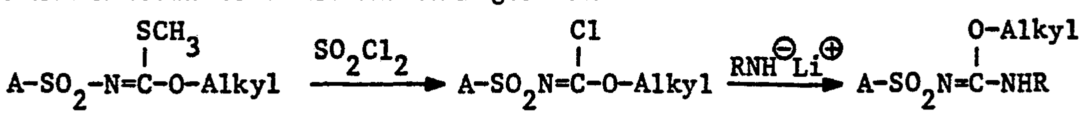


Diese Synthese ist wegen ihrer Abhängigkeit von Sulfonamiden als Ausgangsmaterial und wegen ihrer Aufwendigkeit für die Herstellung von Isoharnstoffen nicht leicht durchführbar. Zudemist das dabei entstehende Methylthiochlorid aus ökologischen Gründen unerwünscht.

In einer bevorzugten Ausführungsform des erfindungsgemässen Verfahrens wird so vorgegangen, dass man ein Sulfonylchlorid der Formel II mit dem Imidkohlensäurediäthylester in Gegenwart eines tertiären Amines in einem organischen Lösungsmittel reagieren lässt, den erhaltenen Sulfonylimidokohlensäureester der Formel IV durch Eindampfen der Mutterlauge isoliert und, falls nötig durch Chromatographie über eine Silikagelsäule reinigt und dann in einem ätherartigen Lösungsmittel in Gegenwart von Natriumhydrid oder Kalium-tert.-Butylat mit einem 2-Aminotriazinyl oder 2-Aminopyrimidin der Formel V reagieren lässt, den Sulfonylisoharnstoff dei Formel V durch Eindampfen und Waschen mit verdünnter Säure isoliert und als Rohprodukt weiter durch Erwärmen in einem organischen Lösungsmittel mit Salzsäure bei 50°C zum Sulfonylharnstoff der Formel I umsetzt und diesen als solchen oder als Salz isoliert. Die Salzsäure kann je nachdem, ob das Lösungsmittel mit Wasser mischbar ist oder nicht, als wässrige Lösung oder gasförmig

eingesetzt werden.

Die Ausgangsverbindungen der Formel II sind bekannt oder können nach bekannten Methoden hergestellt werden.

Die Sulfonylimidocarbonsäureester der Formel IV sowie gewisse Isoharnstoffe der Formel VI sind neu und wurden speziell zur Durchführung des erfindungsgemässen Verfahrens entwickelt. Sie bilden deshalb einen weiteren Gegenstand der vorliegenden Erfindung.

Die neuen Sulfonylimido-kohlensäure-diester entsprechen der Formel IV

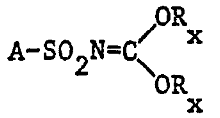

(IV).,

worin A die unter Formel I gegebene Bedeutung hat, $R_1$ aber nicht Wasserstoff sein darf, und $R_x$ $C_1$-$C_5$ Alkyl bedeutet.

Neu sind diejenigen Sulfonylisoharnstoffe der Formel VIa

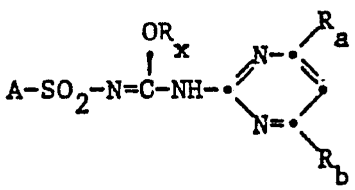

(VI a),

worin $R_a$ den Difluormethoxyrest bedeutet während A, $R_b$ und $R_x$ die oben gegebene Bedeutung haben, sowie die Phenylsulfonylisoharnstoffe der Formel VII

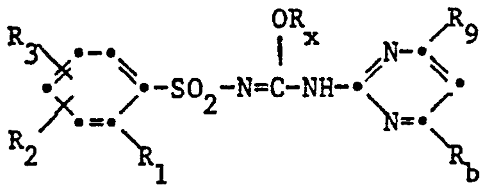

(VII) ,

worin $R_1$ einen Rest $-XR_{11}$ bedeutet während $R_2$, $R_3$, $R_a$, $R_b$, $R_x$ und E die oben gegebene Bedeutung haben.

Die folgenden Beispiele dienen der näheren Erläuterung der Erfindung.

**Beispiel I:**

Herstellung von N-(2-Difluormethoxybenzolsulfonyl)-imido-kohlensäure-diäthylester

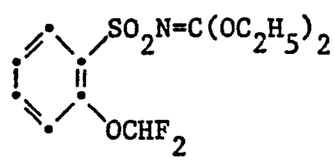

Smp. 80-81°C

Zu einer Lösung von 24,3 g (0,1 Mol) 2-Difluormethoxy-benzolsulfonyl-chlorid und 11,7 g (0,1 Mol) Imidokohlensäure-diäthylester in 70 ml Aceton werden unter Rühren bei Raumtemperatur 11,1 g (0,11 Mol) Triäthylamin getropft. Die Reaktion verläuft leicht exotherm. Man rührt einige Stunden bei Raumtemperatur und filtriert dann das Triäthylamin-Hydrochlorid ab. Die Mutterlauge wird eingedampft, das zurückbleibende Öl mittels Chloroform/Äther/Petroläther 3:3:2 an einer Silicagelsäure chromatographiert. Nach Verdampfen des Eluates verbleiben 23 g des obigen Esters, welcher kristallisiert (72% d.Th), Schmelzpunkt 80-81°C.

Analyse: Ber. C 44,58% H 4,68% N 4,33% S 9,92% F 11,75%

Gef. C 44,5% H 4,6% N 4,3% S 10% F 11,9%.

Analog zu diesem Beispiel werden folgende Benzolsulfonyl-imidokohlensäureester erhalten:

**Tabelle 1**

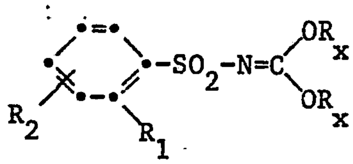

| $R_1$ | $R_2$ | $R_x$ | physikalische Daten |
|---|---|---|---|
| $-COOCH_3$ | H | $C_2H_5$ | Smp: 92-94°C |
| $-COOCH_3$ | H | $CH_3$ | |
| $-COOCH_3$ | H | $C_4H_9$ | |
| -Cl | H | $C_2H_5$ | Smp: 73-75°C |
| -Cl | H | $CH_3$ | |
| -Cl | H | $C_6H_{13}$ | |
| $-COOC_2H_5$ | H | $C_2H_5$ | |
| $-CONH_2$ | H | $C_2H_5$ | |
| $-CONHCH_3$ | H | $C_2H_5$ | |
| $-CON(CH_3)_2$ | H | $C_2H_5$ | |
| -COH | H | $C_2H_5$ | |

**Tabelle 1** (Fortsetzung)

| $R_1$ | $R_2$ | $R_x$ | physikalische Daten |
|---|---|---|---|
| $-COCH_3$ | H | $C_2H_5$ | |
| -F | H | $C_2H_5$ | |
| -Br | H | $C_2H_5$ | |
| $-C{\equiv}N$ | H | $C_2H_5$ | Smp: 78-80°C |
| $-NO_2$ | H | $CH_3$ | |
| $-NO_2$ | H | $C_2H_5$ | |
| $-CF_3$ | H | $C_2H_5$ | |
| $-CH_3$ | H | $C_2H_5$ | |
| $-OCH_3$ | H | $C_2H_5$ | |
| $-SCH_3$ | H | $C_2H_5$ | |
| $-SO_2CH_3$ | H | $C_2H_5$ | |
| $-SOCH_3$ | H | $C_2H_5$ | |
| $-SO_2CH(CH_3)_2$ | H | $C_2H_5$ | |
| $-SO_2C_3H_7$-n | H | $C_2H_5$ | |
| $-SO_2N(CH_3)_2$ | H | $C_2H_5$ | |
| $-OCHF_2$ | H | $CH_3$ | |
| $-OCHF_2$ | H | $C_2H_5$ | Smp: 80-81°C |
| $-OCF_3$ | H | $C_2H_5$ | |
| $-OC_2F_5$ | H | $C_2H_5$ | |
| $-OCF_2CHF_2$ | H | $C_2H_5$ | |
| $-OCH_2CH_2OCH_2$ | H | $C_2H_5$ | |
| $-OCH_2CH_2Cl$ | H | $C_2H_5$ | |

**Tabelle 1** (Fortsetzung)

| $R_1$ | $R_2$ | $R_x$ | physikalische Daten |
|---|---|---|---|
| $-OCCl=CHCl$ | H | $C_2H_5$ | |
| $-OCH_2CH=CH_2$ | H | $C_2H_5$ | |
| $-OCH_2C\equiv CH$ | H | $C_2H_5$ | |
| $-SCHF_2$ | H | $C_2H_5$ | Smp: 72-74°C |
| $-OCHF_2$ | $5-OCHF_2$ | $C_2H_5$ | |
| $-Cl$ | 6-Cl | $C_2H_5$ | |
| $-OCH_3$ | $5-OCH_3$ | $C_2H_5$ | |
| $-OCHF_2$ | 6-Cl | $C_2H_5$ | |

**Tabelle 2**

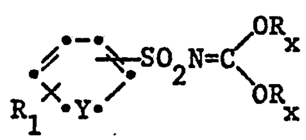

| $R_1$ | $R_x$ | Y | Stellung der Sulfonylgruppe | phys. Daten |
|---|---|---|---|---|
| $2-COOCH_3$ | $C_2H_5$ | S | 3 | |
| $3-COOCH_2$ | $C_2H_5$ | S | 2 | |
| $4-COOCH_3$ | $C_2H_5$ | S | 3 | |
| $2-NO_2$ | $C_2H_5$ | S | 3 | |
| $3-NO_2$ | $C_2H_5$ | S | 2 | |

**Tabelle 2** (Fortsetzung)

| $R_1$ | $R_x$ | Y | Stellung der Sulfonylgruppe | phys. Daten |
|---|---|---|---|---|
| 2-Cl | $C_2H_5$ | S | 3 | |
| 3-Cl | $C_2H_5$ | S | 2 | |
| $2-SO_2N(CH_3)_2$ | $C_2H_5$ | S | 3 | |
| $3-SO_2N(CH_3)_2$ | $C_2H_5$ | S | 2 | |
| $2-SO_2CH_3$ | $C_2H_5$ | S | 3 | |
| $3-SO_2CH_3$ | $C_2H_5$ | S | 2 | |
| H | $C_2H_5$ | O | 2 | |
| H | $C_2H_5$ | C=N | 2 | |
| 2-Cl | $C_2H_5$ | C=N | 3 | |
| $2-OCH_3$ | $C_2H_5$ | C=N | 3 | |
| 2-F | $C_2H_5$ | C=N | 3 | |
| $2-SO_2CH_3$ | $C_2H_5$ | C=N | 3 | |

**Tabelle 3**

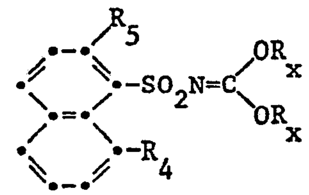

| $R_4$ | $R_5$ | $R_x$ | phys. Daten |
|---|---|---|---|
| H | H | $C_2H_5$ | |
| Cl | H | $C_2H_5$ | |
| Cl | Cl | $C_2H_5$ | |
| $CH_3$ | H | $C_2H_5$ | |
| $CH_3$ | Cl | $C_2H_5$ | |
| $OCH_3$ | $CH_3$ | $C_2H_5$ | |

**Beispiel 2:**

Herstellung von N-(2-Difluormethoxy-benzolsulfonyl)-N'-(4-methoxy-6-methylpyrimidin-2-yl)-O-äthyl-isoharnstoff

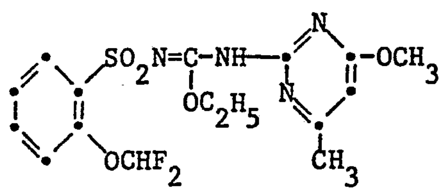

Zu einer Lösung von 11,2 g (0,1 Mol) Kalium-tert.-Butylat in 125 ml Tetrahydrofuran gibt man bei Raumtemperatur unter Rühren 13,9 g (0,1 Mol) 2-Amino-4-methoxy-6-methylpyrimidin. Man rührt während 2 Stunden bei Raumtemperatur und gibt dann 32,3 g (0,1 Mol) N-(2-Difluormethoxy-benzolsulfonyl)-imidokohlensäurediäthylester (erhalten gemäss Beispiel 1) zu und rührt das Reaktionsgemisch während weiteren 20 Stunden. Dann wird eingedampft, der Rückstand in Wasser aufgenommen und mit Salzsäure sauer gestellt. Die saure Lösung wird mit Äthylacetat extrahiert, die organischen Phasen gesammelt, getrocknet und eingedampft. Es bleibt ein fester Rückstand, der mit Äther aufgeschlämmt und dann abfiltriert wird. Man erhält so 34,5 g (83% der Theorie) des obigen Sulfonylisoharnstoffes, welcher bei 131-133°C schmilzt.

Analyse: Ber. C 46,15% H 4,36% N 13,46% S 7,70% F 9,13%

Gef. C 46,4% H 4,4% N 13,4% S 7,7% F 9,2%.

Analog zu diesem Beispiel werden folgende Isoharnstoffe erhalten:

**Tabelle 4**

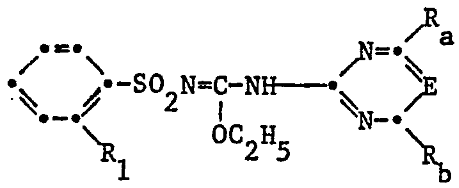

| $R_1$ | $R_a$ | $R_b$ | E | physikalishe Daten |
|---|---|---|---|---|
| $-COOCH_3$ | $-OCHF_2$ | $-CH_3$ | CH | |
| $-COOCH_3$ | $-OCHF_2$ | $-OCH_3$ | CH | |
| $-COOCH_3$ | $-OCHF_2$ | $-Cl$ | CH | |
| $-COOCH_2$ | $-OCHF_2$ | $-N(CH_3)_2$ | CH | |
| $-COOCH_3$ | $-OCHF_2$ | $-CHF_2$ | CH | |
| $-COOCH_3$ | $-OCHF_2$ | $-CF_3$ | CH | |
| $-OCHF_2$ | $-CH_3$ | $-OCH_3$ | N | |
| $-OCHF_2$ | $-OCH_3$ | $-OCH_3$ | N | Smp: 83-87°C |
| $-OCHF_2$ | $-CH_3$ | $-CH_3$ | CH | Smp: 112-114°C |
| $-OCHF_2$ | $-CH_3$ | $-OCH_3$ | CH | Smp: 131-133°C |
| $-OCHF_2$ | $-OCHF_2$ | $-CH_3$ | CH | |
| $-OCHF_2$ | $-OCHF_2$ | $-OCH_3$ | CH | |
| $-OCH_2CH_2OCH_2$ | $-CH_3$ | $-OCH_3$ | N | |
| $-OCH_2CH_2OCH_3$ | $-OCH_3$ | $-OCH_3$ | N | |
| $-OCH_2CH_2OCH_2$ | $-CH_3$ | $-CH_3$ | CH | |
| $-OCH_2CH_2OCH_3$ | $-CH_3$ | $-OCH_3$ | CH | |
| $-OCH_2CH_2OCH_3$ | $-OCHF_2$ | $-CH_3$ | CH | |
| $-OCH_2CH_2OCH_3$ | $-OCHF_2$ | $-OCH_3$ | CH | |
| $-SCHF_2$ | $-CH_3$ | $-OCH_3$ | N | |

**Tabelle 4** (fortsetzung)

| $R_1$ | $R_a$ | $R_b$ | E | physikalische Daten |
|---|---|---|---|---|
| $-SCHF_2$ | $-OCH_3$ | $-OCH_3$ | N | |
| $-SCHF_2$ | $-CH_3$ | $-CH_3$ | CH | |
| $-SCHF_2$ | $-CH_3$ | $-OCH_3$ | CH | |
| $-SCHF_2$ | $-OCHF_2$ | $-CH_3$ | CH | |
| $-SCHF_2$ | $-OCHF_2$ | $-OCH_3$ | CH | |
| $-OCH_2CH_2Cl$ | $-CH_3$ | $-OCH_3$ | N | |
| $-OCH_2CH_2Cl$ | $-OCH_3$ | $-OCH_3$ | N | |
| $-OCH_2CH_2Cl$ | $-CH_3$ | $-CH_3$ | CH | |
| $-OCH_2CH_2Cl$ | $-CH_3$ | $-OCH_3$ | CH | |
| $-OCH_2CH_2Cl$ | $-OCHF_2$ | $-CH_3$ | CH | |
| $-OCH_2CH_2Cl$ | $-OCHF_2$ | $-OCH_3$ | CH | |
| $-OCCl=CHCl$ | $-CH_3$ | $-OCH_3$ | N | |
| $-OCCl=CHCl$ | $-OCH_3$ | $-OCH_3$ | N | |
| $-OCCl=CHCl$ | $-CH_3$ | $-CH_3$ | CH | |
| $-OCCl=CHCl$ | $-CH_3$ | $-OCH_3$ | CH | |
| $-OCCl=CHCl$ | $-OCHF_2$ | $-CH_3$ | CH | |
| $-OCCl=CHCl$ | $-OCHF_2$ | $-OCH_3$ | CH | |
| $-OCH=CH_2$ | $-CH_3$ | $-OCH_3$ | N | |
| $-OCH=CH_2$ | $-OCH_3$ | $-OCH_3$ | N | |
| $-OCH=CH_2$ | $-CH_3$ | $-CH_3$ | CH | |
| $-OCH=CH_2$ | $-CH_3$ | $-OCH_3$ | CH | |
| $-OCH=CH_2$ | $-OCHF_2$ | $-CH_3$ | CH | |

**Tabelle 4** (Fortsetzung)

| $R_1$ | $R_a$ | $R_b$ | E | physikalische Daten |
|---|---|---|---|---|
| $-OCH=CH_2$ | $-OCHF_2$ | $-OCH_3$ | CH | |
| $-OCF_3$ | $-CH_3$ | $-OCH_3$ | N | |
| $-OCF_3$ | $-OCH_3$ | $-OCH_3$ | N | |
| $-OCF_3$ | $-CH_3$ | $-CH_3$ | CH | |
| $-OCF_3$ | $-CH_3$ | $-OCH_3$ | CH | |
| $-OCF_3$ | $-OCHF_2$ | $-CH_3$ | CH | |
| $-OCF_3$ | $-OCHF_2$ | $-OCH_3$ | CH | |
| $-OC_2F_5$ | $-CH_3$ | $-OCH_3$ | N | |
| $-OC_2F_5$ | $-OCH_3$ | $-OCH_3$ | N | |
| $-OC_2F_5$ | $-CH_3$ | $-CH_3$ | CH | |
| $-OC_2F_5$ | $-CH_3$ | $-OCH_3$ | CH | |
| $-OC_2F_5$ | $-OCHF_2$ | $-CH_3$ | CH | |
| $-OC_2F_5$ | $-OCHF_2$ | $-OCH_3$ | CH | |
| $-Cl$ | $-OCHF_2$ | $-OCH_3$ | CH | |
| $-Cl$ | $-OCHF_2$ | $-N(CH_3)_2$ | CH | |
| $-Cl$ | $-OCHF_2$ | $-CH_3$ | CH | |
| $-COOCH_3$ | $-CH_3$ | $-CH_3$ | CH | Smp. 85-88° |
| $-COOCH_3$ | $-CH_3$ | $-OCH_3$ | CH | Smp. 96-98° |
| $-CONH$ | $-OCHF_2$ | $-OCH_3$ | CH | |
| $-CONH_2$ | $-OCHF_2$ | $-N(CH_3)_2$ | CH | |
| $-CONH_2$ | $-OCHF_2$ | $-CH_3$ | CH | |
| $-CONHCH_3$ | $-OCHF_2$ | $-OCH_3$ | CH | |

**Tabelle 4** (Fortsetzung)

| $R_1$ | $R_a$ | $R_b$ | E | physikalische Daten |
|---|---|---|---|---|
| $-CONHCH_3$ | $-OCHF_2$ | $-N(CH_3)_2$ | CH | |
| $-CONHCH_3$ | $-OCHF_2$ | $-CH_3$ | CH | |
| $-CON(CH_3)_2$ | $-OCHF_2$ | $-OCH_3$ | CH | |
| $-CON(CH_3)_2$ | $-OCHF_2$ | $-N(CH_3)_2$ | CH | |
| $-CON(CH_3)_2$ | $-OCHF_2$ | $-CH_3$ | CH | |
| $-NO_2$ | $-OCHF_2$ | $-OCH_3$ | CH | |
| $-NO_2$ | $-OCHF_2$ | $-N(CH_3)_2$ | CH | |
| $-NO_2$ | $-OCHF_2$ | $-CH_3$ | CH | |
| $-CF_3$ | $-OCHF_2$ | $-OCH_3$ | CH | |
| $-CF_3$ | $-OCHF_2$ | $-N(CH_3)_2$ | CH | |
| $-CF_3$ | $-OCHF_2$ | $-CH_3$ | CH | |
| $-OCH_3$ | $-OCHF_2$ | $-OCH_3$ | CH | |
| $-OCH_3$ | $-OCHF_2$ | $-N(CH_3)_2$ | CH | |
| $-OCH_3$ | $-OCHF_2$ | $-CH_3$ | CH | |
| $-SCH_3$ | $-OCHF_2$ | $-OCH_3$ | CH | |
| $-SCH_3$ | $-OCHF_2$ | $-N(CH_3)_2$ | CH | |
| $-SCH_3$ | $-OCHF_2$ | $-CH_3$ | CH | |
| $-SC_3H_7-n$ | $-OCHF_2$ | $-OCH_3$ | CH | |
| $-SC_3H_7-n$ | $-OCHF_2$ | $-N(CH_3)_2$ | CH | |
| $-SC_3H_7-n$ | $-OCHF_2$ | $-CH_3$ | CH | |

**Beispiel 3:**

Herstellung von N-(2-Difluormethyl-benzolsulfonyl)-N'-(4,6-dimethyl-pyrimidin-2-yl)-harnstoff

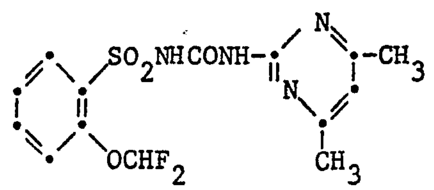

Zu einer Lösung von 10 g Chlorwasserstoff in 100 ml Dioxan gibt man unter Rühren bei Raumtemperatur 8,0 g (0,02 Mol) N-(2-Difluormethoxy-benzolsulfonyl)-N'-(4,6-dimethyl-pyrimidin-2-yl)-O-äthyl-isoharnstoff (erhalten gemäss Beispiel 2). Das Reaktionsgemisch wird dann bei 50°C während 12 Stunden gerührt. Dann dampft man ein, schlämmt die zurückbleibende feste Masse mit Wasser auf und filtriert. Man erhält so 7,2 g (97% d.Th.) des obigen Harnstoffes vom Schmelzpunkt 190-195°C.

Analyse: Ber. C 45,16% H 3,79% N 15,05% S 8,61% F 10,21%

Gef. C 44,8% H 3,9% N 15% S 8,6% F 10,2%.

Gemäss diesem Beispiel können die Isoharnstoffe der Tabelle 4 umgesetzt werden.

**Patentansprüche**

1. Verfahren zur Herstellung von herbizid wirksamen und den Pflanzenwuchsregulierenden Sulfonylharnstoffen der Formel I

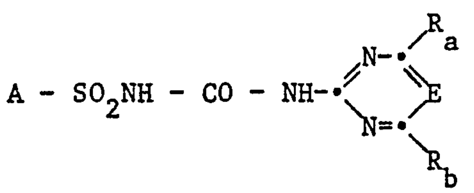 (I) ,

worin A einen Rest der Formeln 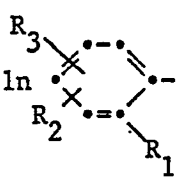 , 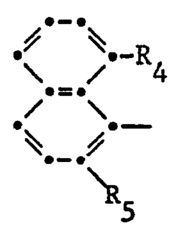 oder 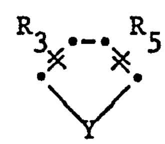

Y Sauerstoff, Schwefel oder 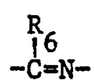,

$R_1$ Wasserstoff, Halogen, Nitro, Trifluormethyl, $C_1$-$C_5$ Alkyl, $C_1$-$C_5$ Alkoxy, -$COR_7$, -$S(O)_m$-$C_1$-$C_5$ Alkyl,-$SO_2R_{10}$ oder $XR_{11}$,-$OSO_2C_1$-$C_5$ Alkyl,

$R_2$ Wasserstoff, Fluor, Chlor, Brom, Nitro, $C_1$-$C_5$ Alkyl, $C_1$ $C_5$ Alkoxy, Trifluormethyl $C_1$-$C_5$ Halogenalkoxy oder -$COR_7$,

$R_3$ Wasserstoff, Fluor, Chlor, Brom, Nitro, Methoxy oder Trifluormethyl,

$R_4$ Wasserstoff, Halogen, Nitro, $C_1$-$C_5$ Alkyl, Methoxy, -$COR_7$ oder -$SO_2NR_8R_9$,

$R_5$ Wasserstoff, Fluor, Chlor, Brom, Nitro, $C_1$-$C_5$ Alkyl, $C_1$-$C_5$ Alkoxy, Trifluormethyl -$S(O)_mC_1$-$C_5$ Alkyl -$COR_7$, -$SO_2NR_8R_9$,

$R_6$ Wasserstoff, Fluor, Methyl, Methoxy,

$R_7$ Wasserstoff, $C_1$-$C_5$ Alkyl, $C_1$-$C_5$ Halogenalkyl, $C_1$-$C_5$ Alkoxy, $C_1$-$C_5$ Halogenalkoxy, $C_2$-$C_{10}$ Alkoxyalkoxy, $C_3$-$C_5$ Alkenyloxy, $C_3$-$C_5$ Alkinyloxy, Phenoxy, Benzyloxy, $C_1$-$C_5$ Alkylthio oder -$NR_8R_9$,

$R_8$ Wasserstoff, $C_1$-$C_5$ Alkyl, Cyanoalkyl mit höchstens 5 Kohlenstoffatomen, Methoxy, Äthoxy oder $C_3$-$C_5$ Alkenyl,

$R_9$ Wasserstoff, $C_1$-$C_5$ Alkyl oder $C_3$-$C_5$ Alkenyl, oder

$R_8$ und $R_9$ zusammen mit dem sie bindenden Stickstoffatom einen 5- bis 6-gliedrigen gesättigten, gegebenenfalls ein Sauerstoff- oder Schwefelatom als Ringglied enthaltenden Heterocyclus,

$R_{10}$ $C_1$-$C_5$ Halogenalkoxy oder -$NR_8R_9$,

$R_{11}$ $C_1$-$C_5$ Alkyl welches substituiert ist durch Halogen, $C_1$ -$C_5$ Alkoxy, -$S(O)_mC_1$-$C_5$ Alkyl, -$S(O)_mC_1$-$C_5$ Halogenalkyl oder $C_2$-$C_5$ Alkenyl welches gegebenenfalls durch einen der obigen Reste substituiert ist,

X Sauerstoff oder -$S(O)_m$,

m eine Zahl Null, Eins oder Zwei,

E die Methingruppe oder Stickstoff,

$R_a$ Wasserstoff, Halogen, $C_1$-$C_5$ Alkyl, $C_1$-$C_5$ Halogenalkyl, $C_1$-$C_4$ Alkoxy, $C_1$-$C_5$ Halogenalkoxy, $C_1$-$C_5$ Alkylthio, $C_2$-$C_{10}$ Alkoxyalkyl oder $C_2$-$C_{10}$ Alkoxyalkoxy,

$R_b$ dasselbe wie $R_a$ oder eine Aminogruppe 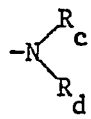 , worin

$R_c$ Wasserstoff, Methyl oder Äthyl und

$R_d$ Wasserstoff, Methyl, Äthyl oder Methoxy, bedeutet, sowie die Salze dieser Verbindungen, dadurch gekennzeichnet, dass man ein Sulfonylchlorid der Formel II

A-$SO_2$ Cl (II)

worin A die oben gegebene Bedeutung hat, in einem inerten Phasensystem oder organischen Lösungsmittel, in Gegenwart der mindestens äquimolaren Menge einer Base als säurebindendes Mittel, bei einer Temperatur von 0° bis 100° C, mit einem Imidokohlensäureester der Formel III

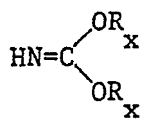

(III)

worin $R_x$ $C_1$-$C_5$ Alkyl bedeutet, kondensiert, den entstandenen Sulfonylimidokohlensäurediester der Formel IV isoliert

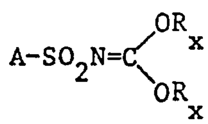

(IV)

und ihn dann in einem inerten Lösungsmittel, in Gegenwart einer starken Base, bei einer Temperatur zwischen 0° und 100° C mit einem 2-Aminopyridin oder 2-Aminotriazin der Formel V

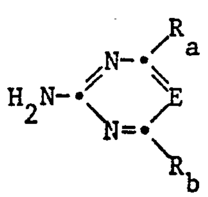

(V)

zu einem Sulfonylisoharnstoff der Formel VI

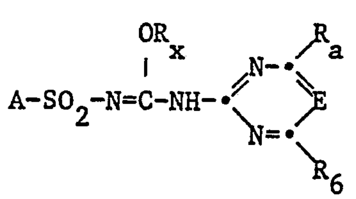

(VI)

umsetzt und diesen anschliessend in Gegenwart eines Halogenwasserstoffes in einem inerten Lösungsmittel bei einer Temperatur von 0 - 100° C in einen Sulfonylharnstoff der Formel I überführt und diesen als solchen oder als Salz isoliert, wobei in den Formeln II-VI A, E, $R_a$, $R_b$ und $R_x$ die oben gegebene Bedeutung haben.

2. Verfahren gemäss Anspruch 1, dadurch gekennzeichnet, dass man das Sulfonylchlorid der Formel II

A - $SO_2$Cl (II)

worin A die im Anspruch 1 gegebene Bedeutung hat, in einem inerten Phasensystem oder organischen Lösungsmittel in Gegenwart eines tertiären Amins mit Imidokohlensäure-diäthylester reagieren lässt, den erhaltenen Sulfonylimido-Kohlensäure der Formel IV

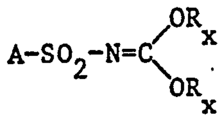
(IV)

worin A und $R_x$ die im Anspruch 1 gegebene Bedeutung haben, in einem wasserfreien Lösungsmittel mit Natriumhydrid oder Kalium-tert.-Butylat und einem 2-Aminopyrimidin oder 2-Aminotriazin der Formel V reagieren lässt

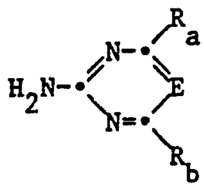
(V)

worin E, $R_a$ und $R_b$ die im Anspruch 1 gegebene Bedeutung haben, den erhaltenen Sulfonylisoharnstoff der Formel VI

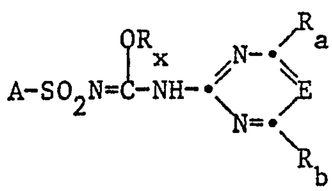
(VI)

worin A, E, $R_a$, $R_b$ und $R_x$ die im Anspruch 1 gegebene Bedeutung haben, in einem organischen Lösungsmittel mit Salzsäure bei 50°C zum Sulfonylharnstoff der Formel I, Anspruch 1 umsetzt und diesen als solchen oder als Salz isoliert.

3. Verfahren gemäss Ansprüchen 1 und 2, dadurch gekennzeichnet, dass man die Umsetzungen der Verbindungen II und III einem inerten Phasengemisch oder organischen Lösungsmittelin Gegenwart von Triäthylamin vorgenommen wird.

4. Verfahren gemäss Ansprüchen 1 und 2, dadurch gekennzeichnet, dass die Umsetzung der Verbindungen IV und V in einem wasserfreien Lösungsmittel in Gegenwart von Kalium-tert. Butylat oder Natriumhydrid vorgenommen wird.

5. Verfahren gemäss Ansprüchen 1 und 2, dadurch gekennzeichnet, dass die Umsetzung des Sulfonylisoharnstoffes der Formel VI mit 10%iger Salzsäure in Dioxan-Lösung bei 50°C vorgenommen wird.

6. Als Zwischenprodukte im Verfahren des Anspruches 1 die neuen Sulfonylimido-Kohlensäure-diester der Formel IV

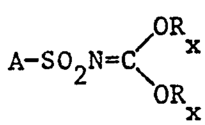
(IV),

worin A die im Anspruch 1 gegebene Bedeutunghat aber $R_1$ nicht Wasserstoff sein darf und $R_x$ $C_1$-$C_5$ Alkyl bedeutet.

7. N-(2-Difluormethoxybenzolsulfonyl)-imidokohlensäurediäthylester gemäss Anspruch 6.

8. N-(2-Chlorbenzolsulfonyl)-imidokohlensäurediäthylester gemäss Anspruch 6.

9. N-(2-Methoxycarbonyl-benzolsulfonyl)-imidokohlensäurediäthylester gemäss Anspruch 6.

10. N-(Difluormethylthio-benzolsulfonyl)-imidokohlensäurediäthylester gemäss Anspruch 6.

11. Als Zwischenprodukt im Verfahren des Anspruches 1 die neuen Sulfonylisoharnstoffe der Formel VIa

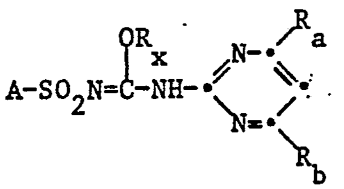 (VIa)

worin $R_a$ den Difluormethoxyrest und A, $R_b$ und $R_x$ die im Anspruch gegebene Bedeutung haben.

12. Als Zwischenprodukte im Verfahren des Anspruches 1 die neuen Phenylsulfonylisoharnstoffe der Formel VII

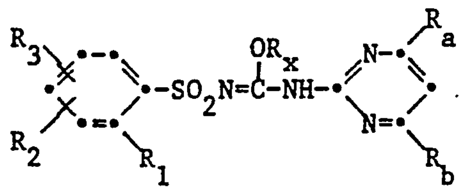 (VII) ,

worin $R_1$ einen Rest $XR_{11}$ bedeutet während $R_2$, $R_3$, $R_a$, $R_b$ und $R_x$ die im Anspruch 1 gegebene Bedeutung haben.

## Claims

1. A process for the preparation of a herbicidal and plant growth regulating sulfonylurea of the formula I

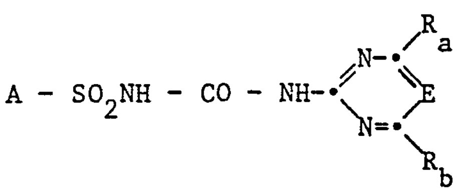 (I)

wherein

A is a radical of the formula

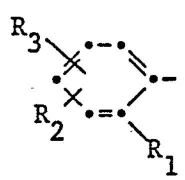 , 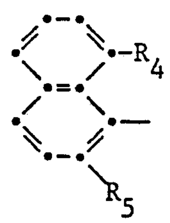 or 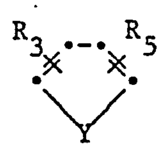

Y is oxygen, sulfur or 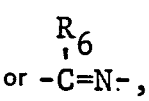 ,

$R_1$ is hydrogen, halogen, nitro, trifluoromethyl, $C_1$-$C_5$-alkyl, $C_1$-$C_5$-alkoxy, -$COR_7$, -$S(O)_m$-$C_1$-$C_5$-alkyl, -$SO_2R_{10}$, $XR_{11}$ or -$OSO_2C_1$-$C_5$-alkyl,

$R_2$ is hydrogen, fluorine, chlorine, bromine, nitro, $C_1$-$C_5$-alkyl, $C_1$-$C_5$-alkoxy, trifluoromethyl, $C_1$-$C_5$-haloalkoxy or -$COR_7$,

$R_3$ is hydrogen, fluorine, chlorine, bromine, nitro, methoxy or trifluoromethyl,

$R_4$ is hydrogen, halogen, nitro, $C_1$-$C_5$-alkyl, methoxy, -$COR_7$ or -$SO_2NR_8R_9$,

$R_5$ is hydrogen, fluorine, chlorine, bromine, nitro, $C_1$-$C_5$-alkyl, $C_1$-$C_5$-alkoxy, trifluoromethyl, -$S(O)_mC_1$-$C_5$-alkyl, -$COR_7$ or -$SO_2NR_8R_9$,

$R_6$ is hydrogen, fluorine, methyl or methoxy,

$R_7$ is hydrogen, $C_1$-$C_5$-alkyl, $C_1$-$C_5$-haloalkyl, $C_1$-$C_5$-alkoxy $C_1$-$C_5$-haloalkoxy, $C_2$-$C_{10}$-alkoxyalkoxy, $C_3$-$C_5$-alkenyloxy, $C_3$-$C_5$-alkynyloxy, phenoxy, benzyloxy, $C_1$-$C_5$-alkylthio or -$NR_8R_9$,

$R_8$ is hydrogen, $C_1$-$C_5$-alkyl, cyanoalkyl having a maximum of 5 carbon atoms, methoxy, ethoxy or $C_3$-$C_5$-alkenyl,

$R_9$ is hydrogen, $C_1$-$C_5$-alkyl or $C_3$-$C_5$-alkenyl, or

$R_8$ and $R_9$ together with the nitrogen atom binding them form a 5- or 6-membered, saturated heterocycle which may contain an oxygen or sulfur atom as ring member,

$R_{10}$ is $C_1$-$C_5$-haloalkoxy or $-NR_8R_9$,

$R_{11}$ is $C_1$-$C_5$-alkyl which is substituted by halogen, $C_1$-$C_5$-alkoxy, $-S(O)_mC_1$-$C_5$-alkyl, $-S(O)_mC_1$-$C_5$-haloalkyl or $C_2$-$C_5$-alkenyl which may be substituted by one of the above radicals,

X is oxygen or $-S(O)_m-$,

m is zero, one or two,

E is the methine group or nitrogen,

$R_a$ is hydrogen, halogen, $C_1$-$C_5$-alkyl, $C_1$-$C_5$-haloalkyl, $C_1$-$C_4$-alkoxy, $C_1$-$C_5$-haloalkoxy, $C_1$-$C_5$-alkylthio, $C_2$-$C_{10}$-alkoxyalkyl or $C_2$-$C_{10}$-alkoxyalkoxy,

$R_b$ is the same as $R_a$ or is an amino group 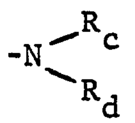 ,

wherein

$R_c$ is hydrogen, methyl or ethyl, and

$R_d$ is hydrogen, methyl, ethyl or methoxy,

or of a salt of such a compound, which process comprises condensing a sulfonyl chloride of the formula II

$A - SO_2Cl$ (II)

wherein A is as defined above, in an inert phase system or organic solvent, in the presence of at least the equimolar amount of a base as acid acceptor and at a temperature in the range from 0° to 100°C, with an imidocarbonic acid ester of the formula III

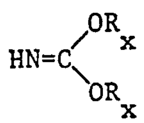

(III)

in which $R_x$ is $C_1$-$C_5$-alkyl; isolating the resultant sulfonylimidocarbonic acid diester of the formula IV

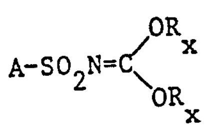

(IV)

then reacting said diester in an inert solvent in the presence of a strong base, at a temperature in the range from 0° to 100°C, with a 2-aminopyridine or 2-aminotriazine of the formula V

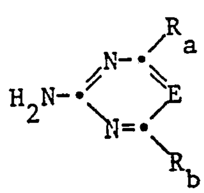

(V)

to give a sulfonylisourea of the formula VI

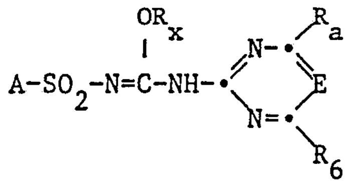 (VI);

and subsequently converting this in the presence of a hydrogen halide in an inert solvent, at a temperature in the range from 0° to 100° C into a sulfonylurea of the formula I; and finally isolating this as such or as a salt; the symbols A, E, $R_a$, $R_b$ and $R_x$ in the formulae II-VI having the meanings defined in the foregoing.

2. A process according to claim 1, which process comprises reacting a sulfonyl chloride of the formula II

$A - SO_2Cl$ (II)

in which A is as defined in claim 1, in an inert phase system or organic solvent, in the presence of a tertiary amine, with the imidocarbonic acid diethyl ester; reacting the resultant sulfonylimidocarbonic acid diester of the formula IV

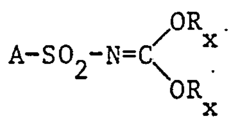 (IV)

in which A and $R_x$ are as defined in claim 1, in an anhydrous solvent, with sodium hydride or potassium tert-butylate and a 2-aminopyrimidine or 2-aminotriazine of the formula V

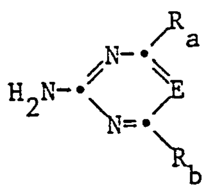 (V)

in which E, $R_a$ and $R_b$ are as defined in claim 1; reacting the resultant sulfonylisourea of the formula VI

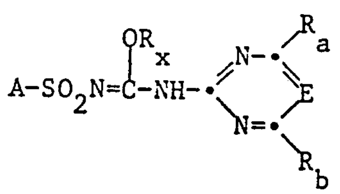 (VI)

in which A, E, $R_a$, $R_b$ and $R_x$ are as defined in claim 1, in an organic solvent with hydrochloric acid at 50° C to obtain the sulfonylurea of the formula I indicated in claim 1; and isolating this as such or as a salt.

3. A process according to either of claims 1 or 2, wherein the reaction of the compounds II and III is performed in an inert phase mixture or organic solvent in the presence of triethylamine.

4. A process according to either of claims 1 or 2, wherein the reaction of the compounds IV and V is performed in an anhydrous solvent in the presence of potassium tert-butylate or sodium hydride.

5. A process according to either of claims 1 or 2, wherein the reaction of the sulfonylisourea of the formula VI with 10 % hydrochloric acid is performed in a dioxane solution at 50° C.

6. As intermediate in the process of claim 1, a novel sulfonylimido-carbonic acid diester of the formula IV

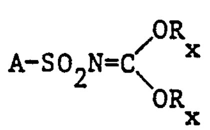 (IV)

in which A is as defined in claim 1, but $R_1$ cannot be hydrogen, and $R_x$ is $C_1$-$C_5$-alkyl.

7. N-(2-Difluoromethoxybenzenesulfonyl)imidocarbonic acid diethyl ester according to claim 6.

8. N-(2-Chlorobenzenesulfonyl)imidocarbonic acid diethyl ester according to claim 6.

9. N-(2-Methoxycarbonylbenzenesulfonyl)imidocarbonic acid diethyl ester according to claim 6.

10. N-(Difluoromethylthiobenzenesulfonyl)imidocarbonic acid diethyl ester according to claim 6.

11. As intermediate in the process of claim 1, a novel sulfonylisourea of the formula VIa

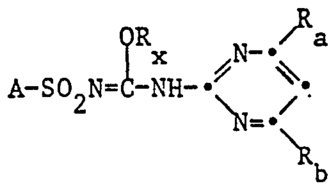

(VIa)

in which $R_a$ is the difluoromethoxy group, and A, $R_b$ and $R_x$ are as defined in claim 1.

12. As intermediate in the process of claim 1, a novel phenylsulfonylisourea of the formula VII

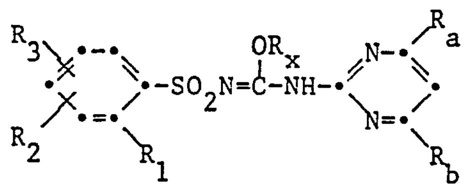

(VII)

in which $R_1$ is a group $XR_{11}$, and $R_2$, $R_3$, $R_a$, $R_b$ and $R_x$ are as defined in claim 1.

## Revendications

1 - Procédé de préparation de sulfonylurées possédant une activité herbicide et régulatrice de la croissance des végétaux et répondant à la formule I

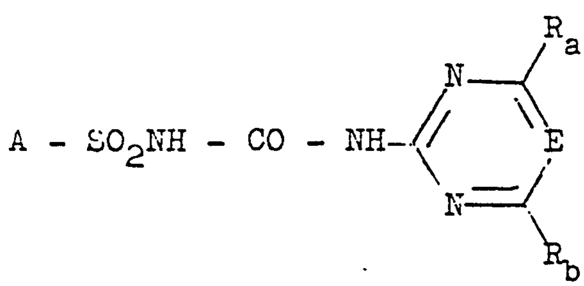

(I),

dans laquelle

A représente un groupe de formule 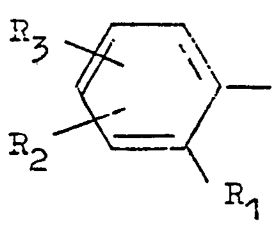 ,

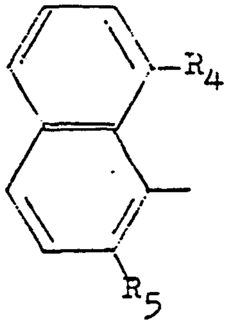

ou

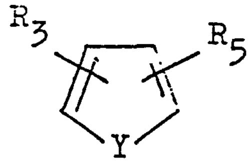

Y représente l'oxygène, le soufre ou le groupe 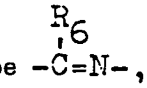 ,

$R_1$ représente l'hydrogène, un halogène, un groupe nitro, trifluorométhyle, alkyle en C 1-C 5, alcoxy en C 1-C 5, $-COR_7$, $-S(O)_m$-alkyle en C 1-C 5, $-SO_2R_{10}$ ou $XR_{11}$, $-OSO_2$alkyle en C 1-C 5,

$R_2$ représente l'hydrogène, le fluor, le chlore, le brome, un groupe nitro, alkyle en C 1-C 5, alcoxy en C 1-C 5, trifluorométhyle, halogénoalcoxy en C 1-C 5 ou $-COR_7$

$R_3$ représente l'hydrogène, le fluor, le chlore, le brome, un groupe nitro, méthoxy ou trifluorométhyle,

$R_4$ représente l'hydrogène, un halogène, un groupe nitro, alkyle en C 1-C 5, méthoxy, $-COR_7$ ou $-SO_2NR_8R_9$,

$R_5$ représente l'hydrogène, le fluor, le chlore, le brome, un groupe nitro, alkyle en C 1-C 5, alcoxy en C 1-C 5, trifluorométhyle, $-S(O)_m$alkyle en C 1-C 5, $-COR_7$, $-SO_2NR_8R_9$,

$R_6$ représente l'hydrogène, le fluor, un groupe méthyl, méthoxy,

$R_7$ représente l'hydrogène un groupe alkyle en C 1-C 5, halogénoalkyle en C 1-C 5 alcoxy en C 1-C 5, halogénoalcoxy en C 1-C 5, alcoxyalcoxy en C 2-C 10, alcényloxy en C 3-C 5, alcynyloxy en C 3-C 5, phénoxy, benzyloxy, alkylthio en C 1-C 5 ou $-NR_8R_9$,

$R_8$ représente l'hydrogène, un groupe alkyle en C 1-C 5, cyanalkyle à 5 atomes de carbone au maximum, méthoxy, éthoxy ou alcényle en C 3-C 5,

$R_9$ représente l'hydrogène, un groupe alkyle en C 1-C 5 ou alcényle en C 3-C 5, ou bien

$R_8$ et $R_9$ forment ensemble et avec l'atome d'azote qui les relie un hétérocycle saturé à 5-6 chaînons contenant éventuellement un atome d'oxygène ou de soufre en tant que chaînon,

$R_{10}$ représente un groupe halogénoalcoxy en C 1-C 5 ou $-NR_8R_9$,

$R_{11}$ représente un groupe alkyle en C 1-C 5 substitué par des halogènes, des groupes alcoxy en C 1-C 5, $-S(O)_m$alkyle en C 1-C 5, $-S(O)_m$halogénoalkyle en C 1-C 5 ou un groupe alcényle en C 2-C 5 portant éventuellement l'un des substituants ci-dessus,

X représente l'oxygène ou $-S(O)_m-$,

m est égal à 0, 1 ou 2,

E représente le groupe méthine ou l'azote,

$R_a$ représente l'hydrogène, un halogène, un groupe alkyle en C 1-C 5, halogénoalkyle en C 1-C 5, alcoxy en C 1-C 4, halogénoalcoxy en C 1-C 5, alkylthio en C 1-C 5, alcoxyalkyle en C 2-C 10 ou alcoxyalcoxy en C 2-C 10,

$R_b$ a les mêmes significations que $R_a$ ou représente un groupe amino 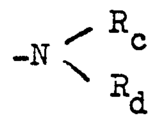 dans lequel

$R_c$ représente l'hydrogène, un groupe méthyle ou éthyle, et

$R_d$ représente l'hydrogène, un groupe méthyle, éthyle ou méthoxy,

et des sels de ces composés, caractérisé en ce que l'on condense un chlorure de sulfonyle de formule II

$A - SO_2Cl$ (II)

dans laquelle A a les significations indiquées ci-dessus, dans un système de phases inerte ou dans un solvant organique inerte, en présence de la quantité au moins équimoléculaire d'une base servant de capteur d'acides, à une température de 0 à 100° C, avec un ester imidocarbonique de formule III

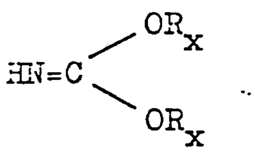

(III)

dans laquelle $R_x$ représente un groupe alkyle en C 1-C 5, on isole le diester sulfonylimidocarbonique formé, de formule IV

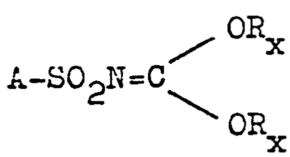

(IV)

et on le convertit ensuite dans un solvant inerte, en présence d'une base forte, à une température de 0 à 100° C, par réaction avec une 2-amino-pyridine ou une 2-amino-triazine de formule V

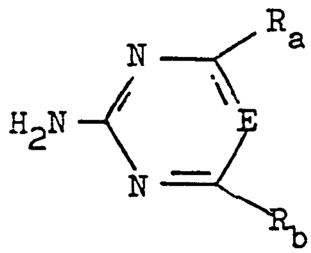

(V)

en une sulfonylisourée de formule VI

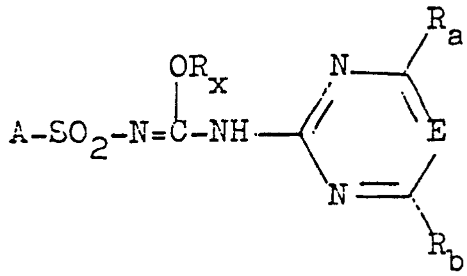

(VI)

qu'on convertit ensuite en présence d'un hydracide halogéné dans un solvant inerte, à une température de 0 à 100° C, en une sulfonylurée de formule I qu'on isole telle quelle ou à l'état de sel, les symboles A, E, $R_a$, $R_b$ et $R_x$ ayant dans les formules II à VI les significations indiquées ci-dessus.

2 - Procédé selon la revendication 1, caractérisé en ce que l'on fait réagir le chlorure de sulfonyle de formule II

A - $SO_2$ Cl (II)

dans laquelle A a les significations indiquées dans la revendication 1, dans un système de phases inerte ou dans un solvant organique inerte, en présence d'une amine tertiaire, avec l'imidocarbonate de diéthyle, ce qui donne l'acide sulfonylimido-carbonique de formule IV

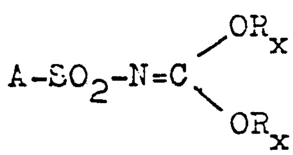

(IV)

dans laquelle A et $R_x$ ont les significations indiquées dans la revendication 1, qu'on fait réagir dans un solvant anhydre avec l'hydrure de sodium ou le tert.-butylate de potassium et une 2-amino-pyrimidine ou 2-amino-triazine de formule V

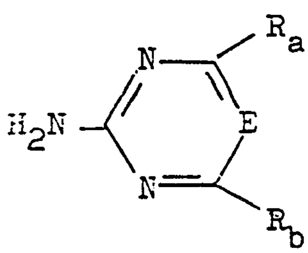

(V)

dans laquelle E, $R_a$ et $R_b$ ont les significations indiquées dans la revendication 1, ce qui donne une sulfonylisourée de formule VI

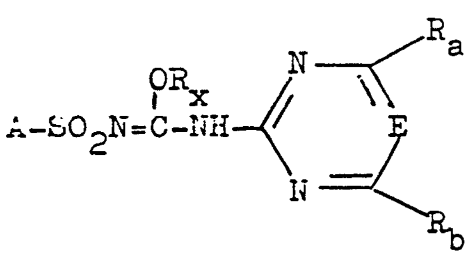

(VI)

dans laquelle A, E, $R_a$, $R_b$ et $R_x$ ont les significations indiquees dans la revendication 1, qu'on convertit dans un solvant organique, à l'aide de l'acide chlorhydrique à 50° C en la sulfonylurée de formule I, revendication 1,

qu'on isole telle quelle ou à l'état de sel.

3 - Procédé selon les revendications 1 et 2, caractérisé en ce que les réactions des composés II et III sont effectuées dans un mélange de phases inerte ou dans un solvant organique inerte en présence de triéthylamine.

4 - Procédé selon les revendications 1 et 2, caractérisé en ce que la réaction des composés IV et V est effectuée dans un solvant anhydre en présence de tert.-butylate de potassium ou d'hydrure de sodium.

5 - Procédé selon les revendications 1 et 2, caractérisé en ce que la réaction de la sulfonylisourée de formule VI est effectuée avec de l'acide chlorhydrique à 10 % en solution dans le dioxanne à 50° C.

6 - En tant que produits intermédiaires du procédé de la revendication 1, les nouveaux diesters sulfonylimido-carboniques répondant à la formule IV

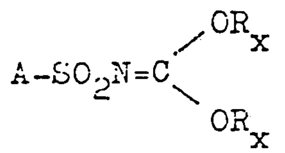

(IV),

dans laquelle A a les significations indiquées dans la revendication 1, $R_1$ toutefois ne pouvant représenter l'hydrogène, et $R_x$ représente un groupe alkyle en C 1-C 5.

7 - Le N-(2-difluorométhoxybenzène-sulfonyl)-imidocarbonate de diéthyle selon la revendication 6.

8 - Le N-(2-chlorobenzène-sulfonyl)-imidocarbonate de diéthyle selon la revendication 6.

9 - Le N-(2-méthoxycarbonyl-benzène-sulfonyl)-imidocarbonate de diéthyle selon la revendication 6.

10 - Le N-(difluorométhylthio-benzène-sulfonyl)-imidocarbonate de diéthyle selon la revendication 6.

11 - En tant que produits intermédiaires du procédé de la revendication 1, les nouvelles sulfonylisourées de formule VIa

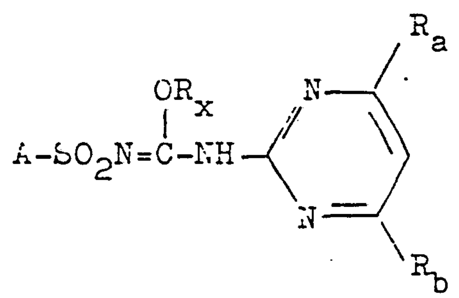

(VIa)

dans laquelle $R_a$ représente le groupe difluorométhoxy et A, $R_b$ et $R_x$ ont les significations indiquées dans la revendication 1.

12 - En tant que produits intermédiaires du procédé de la revendication 1, les nouvelles phénylsulfonylisourées de formule VII

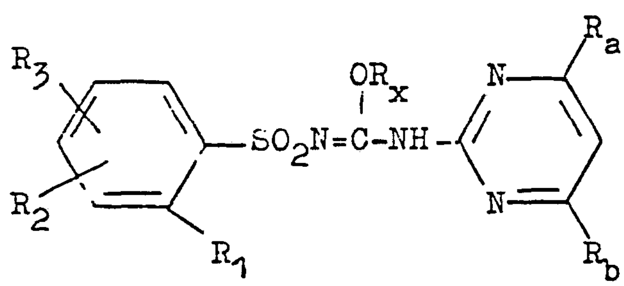

(VII),

dans laquelle $R_1$ représente un groupe $XR_{11}$ et $R_2$, $R_3$, $R_a$, $R_b$ et $R_x$ ont les significations indiquées dans la revendication 1.